# EUROPEAN PATENT SPECIFICATION

(11) **EP 1 853 194 B1**
(45) Date of publication and mention of the grant of the patent: **15.08.2012**
(21) Application number: 06736916.5
(22) Date of filing: 03.03.2006
(51) Int. Cl.: A61C 5/06

(54) **HEATING DENTAL MATERIALS WITH A HEAT PACK**
ERWÄRMUNG VON ZAHNMATERIALIEN MIT WÄRMEPACKUNG
CHAUFFAGE DE MATERIAUX DENTAIRES AVEC UNE POCHE DE CHAUFFAGE

(30) Priority: 04.03.2005 US 658682 P
(43) Date of publication of application: 14.11.2007
(73) Proprietor: DENTSPLY International Inc., York, PA 17405-0872 (US)
(72) Inventor: PIERSON, Paul, R., Camden, DE 19934 (US); HARE, Robert, V., Georgetown, DE 19947 (US)
(74) Representative: Blodig, Wolfgang
(86) International application number: PCT/US2006/007671
(87) International publication number: WO 2006/096536

(56) References cited:
- EP-A- 0 588 304
- US-A- 5 351 675
- US-A- 5 366 491
- US-A- 5 471 767
- US-B1- 6 320 162

## Description

### TECHNICAL FIELD

The present invention generally relates to the delivery of materials such as dental materials. More particularly, the invention relates to heating such materials prior to or during their delivery or other intended use, such as delivery to the oral cavity. Specifically, the invention relates to the use of an exothermic chemical reaction heat pack to warm the materials ad/or the material delivery device or devices.

### BACKGROUND OF THE INVENTION

US-A 6,320,162, discloses a method for preheating dental materials.

In the art of dispensing dental materials, it is common practice to provide the dental professional with devices which are pre-loaded with cements, filling material, restoratives, adhesives, bonding agents, composites, and the like. Often such devices take the form of an ampoule or cartridge, which is inserted into a handheld delivery gun or the like having operating handles or levers and, by operation thereof, desired amounts of the material in the cartridges are discharged readily and precisely. It is often the case in dentistry that relatively small quantities of such materials are used in a given application. Therefore, such materials are often packed in one-time use cartridges in approximately single use amounts or "unit doses".

One example of a cartridge of interest to the present invention comprises the subject matter of prior U.S. Pat. No. 4,391,590 assigned to the assignee of the present invention. In the patented structure, the body of the cartridge is cylindrical and of uniform inner diameter and in which a discharge piston is mounted in position in the open filling end of the cartridge to form both a closure and an ejecting piston. Cartridges of this type are miniature and, by way of example, have an outer diameter of about one quarter inch and an inner diameter of approximately one eighth inch. The piston, accordingly, has a complementary outer diameter of about one eighth inch and at present is composed of solid plastic material. An example of a cartridge heretofore known in the art is sold by DENTSPLY International Inc. of York, PA, under the registered trademark COMPULES.

While such cartridges have enjoyed widespread use, there is always a difficulty in dispensing highly filled and highly viscous dental materials. It is generally known that heating the dental material may help to temporarily reduce its viscosity and assist in the subsequent delivery of the material to the patient. Other dental materials that are not delivered with an ampoule, cartridge, syringe or gun may still benefit from being heated prior to use. For example, heating may not only assist in the delivery of the material by whatever means, but may also initiate, propagate or otherwise assist in polymerization or other reactions. In short, a wide variety of dental materials may benefit from heating for a wide variety of purposes.

An example of a dental material that benefits from heating is described in copending U.S. Pat. App. Ser. No. 60/630495 (published as US 2006229580). The material of that pending application unexpectedly reduces its viscosity at elevated temperatures. For example, the material is extrudable from an ampoule upon reaching 50 to 60 degrees C, it is packable at 35 to 37 degrees C, and it is substantially solid at room temperature.

A variety of other dental materials such as dental composites are currently marketed. These dental composites have a wide range of viscosity. Some of these composites have high viscosity , which makes it difficult for them to be extruded from the primary package , which is often a syringe or ampoule. One way to reduce the extrusion force is to apply heat to the material and package. Warming the material reduces its viscosity and decreases extrusion force.

In the case of the material of U.S. Pat. App. Ser. No. 60/630495, it was found that warming the ampoule containing the material in a microwave oven or with a commercially available ampoule warmer did allow the material to flow as desired. A problem with both of those heating systems is that they require extra equipment that takes up a large amount of counter space and that such equipment is generally expensive to obtain and operate.

A need exists therefore for an efficient and simple device and procedure for heating dental materials.

### SUMMARY OF THE INVENTION

The present invention provides a method of heating a dental material contained in a package as defined by claim 1 and a package as defined by claim 4. According to the present invention, dental materials are heated using exothermic chemical reactions. The reactive components are preferably contained within a package termed a "hot pack". These Hot Packs preferably utilize a dry chemical reaction that produces heat. Any hot pack chemistry that produces heat in the proper range is within the scope of the invention. One useful chemical system comprises iron powder, water, a salt, activated charcoal, and vermiculite. This system is activated by exposure to air, which begins the chemical reaction.

### BRIEF DESCRIPTION OF THE DRAWINGS

Fig. 1 is a front perspective view of a heat pack according to the present invention.

Fig. 2 is an side elevational, partially broken-away view of a exemplary package, namely an ampoule useful for containing a dental material and being of the type conducive to being heated by the heat pack of Fig. 1, for background purposes.

Fig. 3 is a front elevational view of an outer container for the heat pack of Fig. 1.

### PREFERRED EMBODIMENTS FOR CARRYING OUT THE INVENTION

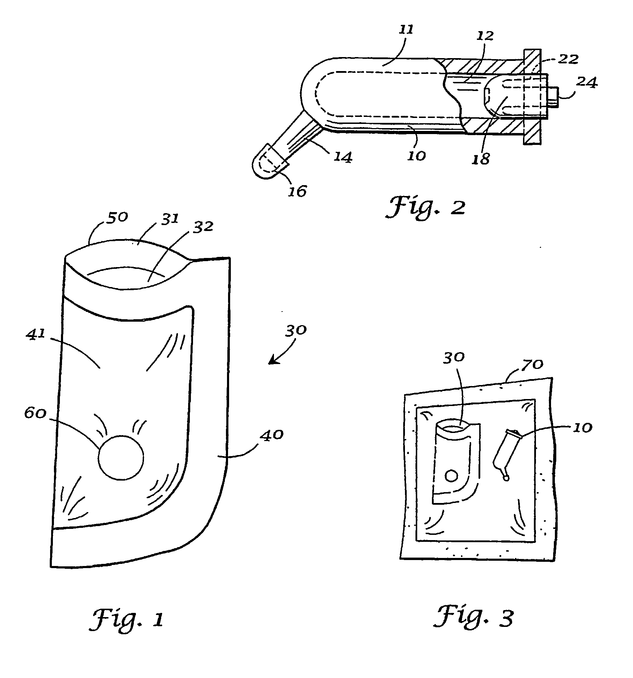

As stated above, it is often useful in the dental arts and in fact many different arts, to heat or warm materials prior to their use. Such materials may benefit from heating for a variety of reasons including for example, to assist in viscosity reduction to promote flow and delivery, to catalyze, initiate or otherwise induce or promote a chemical reaction, and the like. An example of an ampoule useful for containing a dental material is shown for background purposes as ampoule 10 in Fig. 2. Ampoule 10 is of the type having a body member 11 with an internal cavity 12 for containing a dental or other material (the material not being shown). It is provided with a dispensing spout 14 covered by a removable cap 16. After being filled with the dental material, cavity 12 is closed by a moveable piston 18. An operator causes piston 18 to move in a direction toward spout 14, thereby physically pushing material contained within cavity 12 toward spout 14. After cap 16 has been removed, this movement of piston 18 causes the material to be dispensed from spout 14. Ampoule 10 is conventional in the art and is merely one example of a heatable package for a material, in this case a dental material, with which the present invention is useful. Any package that can be heated so as to heat the material contained therein is useful with the present invention, whether it be a box, bag, ampoule, tube, bottle or any other such container without limitation. The container such as ampoule 10 should be conductive of heat sufficiently to allow heat to permeate therethrough and to heat the material contained therein. All such packages and containers will be exemplified herein by dental ampoule 10.

As shown in Fig. 1, an exemplary heat pack according to the concepts of the present invention is generally designated by the number 30. Heat pack 30 is envelope or sock shaped such that it has at least one open end such as end 31. An open cavity 32 is contained within heat pack 30. Open end 31 and cavity 32 are configured so as to receive at least portion and preferably all of ampoule 10 therethrough and therein respectively. Heat pack 30 is preferably of the type that upon exposure to air an exothermic reaction takes place at a predetermined temperature and for a predetermined time, based upon the material being heated.

The shape and construction of heat pack 30 is not necessarily a limitation of the invention. For example, heat pack 30 may be pillow shaped such that the exothermic chemical components are contained therein. Heat pack 30 may also separately hold components that when mixed cause the appropriate reaction. Further, any material of construction for heat pack 30 is within the scope of the invention, such as a spun fiber fabric or other material. Any and all such constructions are within the scope of the invention.

One useful heat pack 30 has is envelope of sock shaped and is formed by a flat sheet being welded at its edges 40. In addition, one or more sides of heat pack 30 may be provided with an at least partially transparent window 41 so that the operator can view the contents of heat pack 30. Further, if desired, open end 31 may be sealed along its edges 50 after insertion of ampoule 10 by any means such as by use of a pressure sensitive adhesive or the like.

It is another aspect of the present invention to provide temperature indicia 60 so that an operator can determine when the proper temperature has been reached or even to determine that the heating has taken place for a sufficient period of time. Any such indicia is within the scope of the present invention. Exemplary indicia 60 is a label that is affixed to heat pack 30. When the appropriate temperature has been reached and/or when the appropriate temperature has been maintained for a predetermined period of time, indicia 60 is induced to provide a visual reference of the same. For example, indicia 60 my change color, a word may appear, it may peal from heat pack 30 or the like. Similarly, heat pack 30 itself may be fabricated from a material that changes color or the like, such that heat pack 30 or a portion thereof itself constitutes and carries out the function of indicia 60. Further still, ampoule 10 may have similar characteristics which would be useful with window 41.

It will be appreciated that envelope-shaped heat pack 30 is merely one example of a heat pack within the scope of the present invention. Other heat packs within the scope of the invention (not shown) could be flat or sheet-like, boxes, bags, bottles, rigid or non-rigid in structure or the like. In addition, a separate non-heating container (not shown) could be provided such that the ampoule 10 and a heat pack are placed therein to induce heating of the ampoule 10. All such constructions are within the scope of the invention and are exemplified by heat pack 30 in the drawings.

If heat pack 30 is of the type wherein the exothermic reaction is initiated upon exposure to air, then it would be necessary to place heat pack 30 into an air-barrier package 70 as shown in Fig. 3. Air-barrier package 10 may be provided for simplicity with a heat pack 30 and an ampoule 10 as one package. In use, the operator would open air-barrier package 70 thus exposing heat pack 30 to the air and initiating the exothermic chemical reaction. Ampoule 10 is then placed into heat pack 30 as above described. Air-barrier package 70 may be fabricated from any suitable material.

In one experiment, a heat pack 30 was prepared containing iron powder, water, a salt, activated charcoal, and vermiculite. Once the exothermic reaction was initiated by exposure to air, the hot pack reached temperatures of from 23 to 63°C within two hours and maintained its temperature and at least 60°C for five hours. After 15 hours, the temperature was still above 45°C. Samples of dental materials were heated using this method and the extrusion force required to extrude the material was found to be acceptable for use in conventional dental procedures. The benefits of the inventive system includes low-cost, minimum space requirements, complete and easy disposability for aseptic concerns, no electricity requirements (therefore, no power cords or the like) and that it is environmentally friendly for disposal.

It should be apparent therefore that a heat pack as shown and described accomplishes the concepts of the present invention. The invention has been exemplified herein and on the drawings without attempting to show all embodiments within the scope thereof The scope of the invention shall be limited only be any attached claims.

## Claims

1. A method of heating a dental material contained in a package, comprising
- providing a heat pack (30) and an ampoule (10) containing a dental material, the heat pack (30) and the ampoule(10) being placed in an air barrier package (70), the heat pack being of the type wherein an exothermic reaction is initiated upon exposure to air;
- opening the air barrier package (70) thus exposing heat pack (30) to air and initiating the exothermic chemical reaction; and
- exposing the package to heat generated by an exothermic chemical reaction within the heat pack by physically contacting the package and said heat pack to assist in viscosity reduction to promote flow and delivery.

2. A method as in claim 1, wherein said exothermic chemical reaction results from the mixture of iron powder, water, a salt, activated charcoal, vermiculite and air.

3. A method as in claim 1, wherein said heat pack is in the shape of an envelope, such that the package may be placed within the envelope.

4. A package comprising an ampoule (10) containing dental material, a heat pack (30) for heating the ampoule(10) and an air barrier package (70), the ampoule (10) and the heat pack (30) being placed in the air barrier package (70), whereby the heat pack (30) comprises a fabric enclosing a mixture of ingredients which can be induced upon exposure to air to react in an exothermic reaction to produce heat.

5. A package as in claim 4, wherein said fabric is configured to receive the packaged dental material.

6. A package as in claim 5 wherein said fabric is in the shape of an envelope having an open end for receiving the packaged dental material.

7. A package as in claim 4, further comprising a temperature indicia.

8. A package as in claim 7 wherein said indicia is in the form of a label affixed to said fabric.

9. A package as in claim 4, wherein said fabric is caused to produce an indicia upon reaching a predetermined temperature.

## Patentansprüche

1. Verfahren zur Erwärmung eines Dentalmaterials, das in einer Verpackung enthalten ist, umfassend
- Bereitstellung einer Wärmepackung (30) und einer Ampulle (10), die ein Dentalmaterial enthält, wobei die Wärmepackung (30) und die Ampulle (10) in eine luftdichte Verpackung (70) eingesetzt sind und die Wärmepackung von demjenigen Typ ist, bei dem eine exotherme Reaktion bei Kontakt mit Luft initiiert wird;
- Öffnen der luftdichten Verpackung (70), um auf diese Weise die Wärmepackung (30) der Luft auszusetzen und eine exotherme chemische Reaktion zu initiieren; und
- Aussetzen der Verpackung der Wärme, die durch eine exotherme chemische Reaktion innerhalb der Wärmepackung erzeugt wird durch physischen Kontakt der Verpackung und der Wärmepackung, um eine Viskositätsreduktion unterstützen, um ein Fließen und eine Abgabe zu fördern.

2. Das Verfahren nach Anspruch 1, wobei die exotherme chemische Reaktion entsteht aus dem Gemisch von Eisenpulver, Wasser, einem Salz, aktivierter Holzkohle, Vermicullit und Luft.

3. Das Verfahren nach Anspruch 1, wobei der die Wärmepackung in Form eines Umschlags vorliegt, wobei die Verpackung in dem Umschlag angeordnet werden kann.

4. Eine Verpackung, umfassend eine Ampulle (10), die ein Dentalmaterial enthält, eine Wärmepackung (30) zum Erwärmen der Ampulle (10) und eine luftdichte Verpackung (70), wobei die Ampulle (10) und die Wärmepackung (30) in der luftdichten Verpackung (70) angeordnet wird, wobei die Wärmepackung (30) ein Gewebe umfasst, das ein Gemisch aus Bestandteilen umschließt, die bei Aussetzen von Luft veranlasst werden können in einer exothermen Reaktion zu reagieren, um Wärme zu erzeugen.

5. Die Packung nach Anspruch 4, wobei das Gewebe ausgestaltet ist, um das verpackte Dentalmaterial zu empfangen.

6. Die Packung nach Anspruch 5, wobei das Gewebe in Form eines Umschlags vorliegt, mit einem offenen Ende zur Aufnahme des verpackten Dentalmaterials.

7. Die Packung nach Anspruch 4, die ferner eine Temperaturanzeige umfasst.

8. Die Packung nach Anspruch 7, wobei die Anzeige in Form eines Labels vorliegt, das auf dem Gewebe befestigt ist.

9. Die Packung nach Anspruch 4, wobei das Gewebe zu einer Anzeige bei Erreichen einer vorbestimmten Temperatur veranlasst wird.

## Revendications

1. Procédé destiné à chauffer du matériau dentaire contenu dans un emballage, comprenant le fait
de fournir un sachet chauffant (30) et une ampoule (10) contenant du matériau dentaire, le sachet chauffant (30) et l'ampoule (10) étant placés dans un emballage barrière à l'air (70), le sachet chauffant étant du type dans lequel une réaction exothermique est initiée lors de l'exposition à l'air ;
d'ouvrir l'emballage barrière à l'air (70) exposant ainsi un sachet chauffant (30) à l'air et initiant la réaction chimique exothermique ; et
d'exposer l'emballage à une chaleur générée par une réaction chimique exothermique dans le sachet chauffant par un contact physique de l'emballage et dudit sachet chauffant pour faciliter la réduction de viscosité afin de favoriser l'écoulement et la livraison.

2. Procédé selon la revendication 1, dans lequel ladite réaction chimique exothermique résulte du mélange de la poudre de fer, de l'eau, du sel, du charbon actif, de la vermiculite et de l'air.

3. Procédé selon la revendication 1, dans lequel ledit sachet chauffant est sous la forme d'une enveloppe, de sorte que l'emballage puisse être placé dans l'enveloppe.

4. Emballage comprenant une ampoule (10) contenant du matériau dentaire, un sachet chauffant (30) destiné à chauffer l'ampoule (10) et un emballage barrière à l'air (70), l'ampoule (10) et le sachet chauffant (30) étant placés dans l'emballage barrière à l'air (70), grâce à quoi le sachet chauffant (30) comprend un tissu entourant un mélange d'ingrédients qui peuvent être induits, lors de l'exposition à l'air, à réagir dans une réaction exothermique pour produire de la chaleur.

5. Emballage selon la revendication 4, dans lequel ledit tissu est configuré pour recevoir le matériau dentaire emballé.

6. Emballage selon la revendication 5, dans lequel ledit tissu est sous la forme d'une enveloppe ayant une extrémité ouverte pour recevoir le matériau dentaire emballé.

7. Emballage selon la revendication 4, comprenant en outre un indice de température.

8. Emballage selon la revendication 7, dans lequel ledit indice est sous la forme d'une étiquette apposée sur ledit tissu.

9. Emballage selon la revendication 4, dans lequel ledit tissu est amené à produire un indice dès qu'une température prédéterminée est atteinte.
